# EUROPEAN PATENT APPLICATION

(11) **EP 4 000 616 A1**
(43) Date of publication of application: **25.05.2022**
(21) Application number: 20306394.6
(22) Date of filing: 17.11.2020
(51) Int. Cl.: A61K 31/381, A61K 31/519, A61P 1/16

(54) **COMBINATION THERAPY FOR THE TREATMENT OF A LIVER DISEASE**

(71) Applicant: Inventiva, 21121 Daix (FR)
(72) Inventor: WETTSTEIN, Guillaume, 21490 NORGES LA VILLE (FR); BROQUA, Pierre, 92160 ANTONY (FR)
(74) Representative: Cabinet Beau de Loménie

(57) **Abstract**

The invention relates to a drug combination and a method for the treatment of a liver disease, the combination comprising lanifibranor and firsocostat.

## Description

### Field of the invention

The present disclosure provides a drug combination and a method for the treatment of a liver disease, in particular for the treatment of non-alcoholic fatty liver disease.

### Background of the invention

Non-alcoholic fatty liver disease (NAFLD) is excessive fat build-up in the liver without another clear cause such as alcohol use. There are two types: non-alcoholic fatty liver (NAFL) and non-alcoholic steatohepatitis (NASH), with the latter also including liver inflammation. NAFLD is the most common liver disorder worldwide and is present in approximately 25% of the world's population (Nutr Clin Pract 2020; Vol.35, n°1, pages 72-84). NAFLD usually does not progress to NASH. However, when NAFLD does progress to NASH, it may eventually lead to complications such as fibrosis, cirrhosis, liver cancer, liver failure, or cardiovascular disease. Because of the devastating complications and comorbidities, NAFLD is a very costly disease for the healthcare system, with estimated annual direct medical costs exceeding $100 billion in the United States alone. Yet, as of today, there is no approved treatment for NAFLD or NASH.

WO 2015/189401 discloses the use of a pan-PPAR agonist, notably 5-Chloro-1-[(6-benzothiazolyl)sulfonyl]-1H-indole-2-butanoic acid (INN: lanifibranor; CAS n° 927961-18-0) for the treatment of a fibrotic condition.

EP-A-3 597 271 discloses a composition comprising an ACC inhibitor having one of the general formulae below: 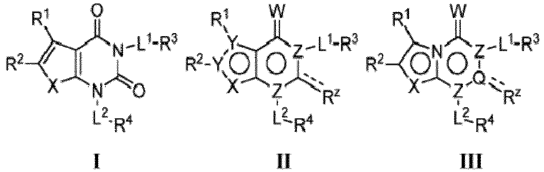 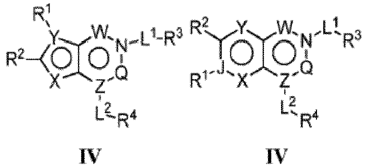 for use in a method of treating, stabilizing, or lessening the severity or progression of a non-alcoholic fatty liver disease comprising administering to a patient in need thereof the composition comprising the ACC inhibitor, optionally wherein the ACC inhibitor is administered in combination with one or more additional therapeutic agents. In some embodiments the ACC inhibitor is: 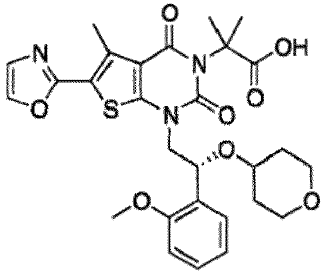 (INN: firsocostat; CAS n° 1434635-54-7). In some embodiments the ACC inhibitor is administered in combination with a PPARα/δ agonist such as GFT505, a PPAKγ agonist such as pioglitazone or a PPARδ agonist. This patent application does not, however, contain any data at all in support of the alleged treatment.

It has now been found that the combined administration of lanifibranor and firsocostat provides an effective treatment against NAFLD.

### Summary of the invention

In one aspect, the present disclosure provides a combination product comprising (i) lanifibranor or a deuterated derivative thereof, and (ii) firsocostat.

In another aspect, the present disclosure provides a combination of lanifibranor (or a deuterated derivative thereof) and firsocostat for use in a method of treating NAFLD or a complication thereof.

In yet another aspect, the present disclosure provides a method of treating NAFLD or a complication thereof, which comprises administering to a subject in need thereof a combination of lanifibranor (or a deuterated derivative thereof) and firsocostat.

### Brief description of the drawings

Figure 1 shows the mean body weight of the mice in each treatment group: vehicle, lanifibranor alone, firsocostat alone and the combination lanifibranor + firsocostat.
Figure 2 shows the mean weight of epididymal white adipose tissue (EWAT) per treatment group.
Figure 3A shows hepatic fatty acid levels per treatment group.
Figure 3B shows hepatic total cholesterol levels per treatment group.
Figure 3C shows hepatic triglycerides levels per treatment group.
Figure 4A shows RNA expression level of IL-1β per treatment group.
Figure 4B shows RNA expression level of MCP-1 per treatment group.
Figure 5A shows RNA expression level of collagen-alphal per treatment group.
Figure 5B shows RNA expression level of TGF-β1 per treatment group.
Figure 6 shows the histological steatosis score per treatment group.
Figure 7 shows the histological inflammation score per treatment group.
Figure 8A shows the histological fibrosis score per treatment group.

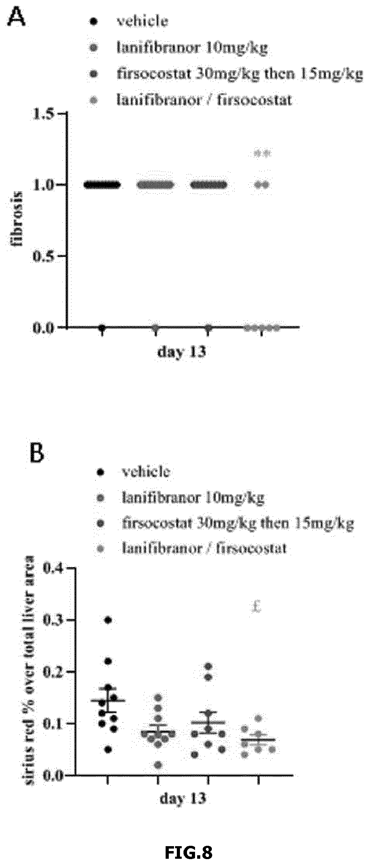

Figure 8A shows the histological fibrosis percentage area per treatment group.

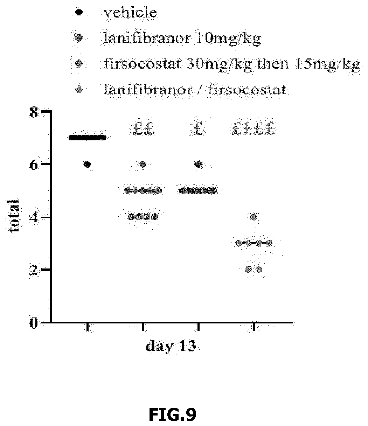

Figure 9 shows the histological total score per treatment group corresponding to the sum of the steatosis, inflammation and fibrosis scores.

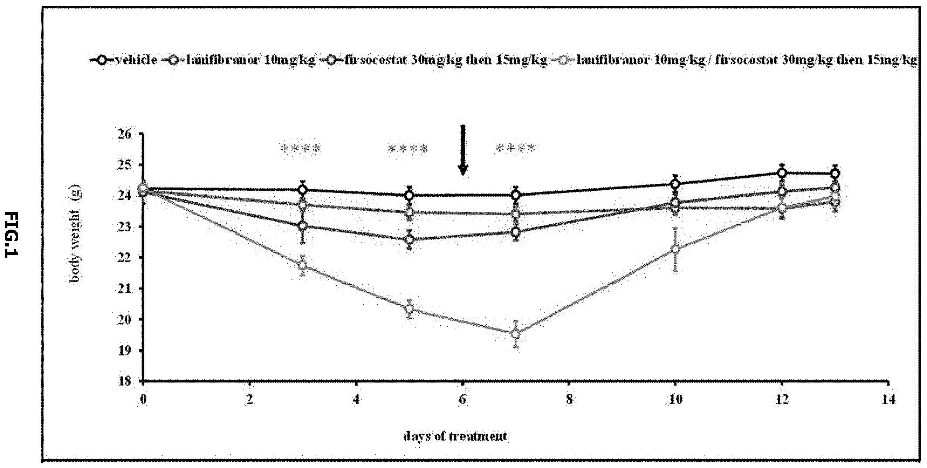

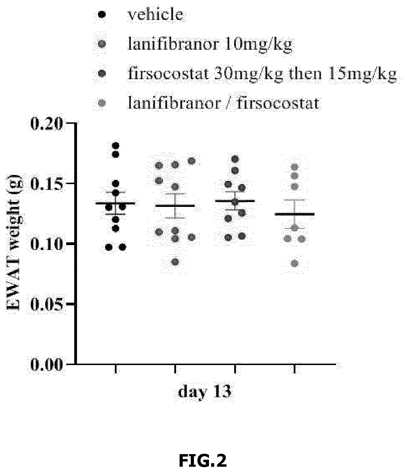

In figure 1 the curves correspond, from top to bottom, to: treatment with vehicle, treatment with lanifibranor, treatment with firsocostat, and treatment with lanifibranor + firsocostat. In figures 2 to 9 the dots correspond, from left to right, to: treatment with vehicle, treatment with lanifibranor, treatment with firsocostat, and treatment with lanifibranor + firsocostat.

### Detailed description of the invention

In one aspect, the present disclosure provides a combination product comprising (i) lanifibranor or a deuterated derivative thereof, and (ii) firsocostat.

In another aspect, the present disclosure provides a combination of (i) lanifibranor (or a deuterated derivative thereof) and (ii) firsocostat for use in a method of treating non-alcoholic fatty liver disease (NAFLD) or a complication thereof.

In yet another aspect, the present disclosure provides a method of treating non-alcoholic fatty liver disease (NAFLD) or a complication thereof, which comprises administering to a subject in need thereof a combination of (i) lanifibranor (or a deuterated derivative thereof) and (ii) firsocostat.

### Definitions

As used herein, "a complication of NAFLD" includes, but is not limited to, steatosis, steatohepatitis, non-alcoholic steatohepatitis (NASH), liver fibrosis caused by NASH, liver cirrhosis caused by NASH, liver failure caused by NASH, cardiovascular disease caused by NASH or hepatocellular carcinoma (HCC) caused by NASH.

The term "subject", as used herein, means a mammal and includes human and animal subjects, such as domestic animals (e.g., horses, dogs, cats, etc.).

The terms "treat" or "treating," as used herein, refers to partially or completely alleviating, inhibiting, delaying onset of, preventing, ameliorating and/or relieving a disease or disorder, or one or more symptoms of the disease or disorder. In some embodiments, treatment may be administered after one or more symptoms have developed. In some embodiments, the term "treating" includes preventing or halting the progression of a disease or disorder. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence. Thus, in some embodiments, the term "treating" includes preventing relapse or recurrence of a disease or disorder.

In the context of the present disclosure, the various embodiments described herein can be combined.

As mentioned above, the present disclosure provides in one aspect a combination product comprising (i) lanifibranor or a deuterated derivative thereof, and (ii) firsocostat, or a pharmaceutically acceptable salt thereof.

The present disclosure also provides a combination of lanifibranor (or a deuterated derivative thereof) and firsocostat for use of in a method of treating non-alcoholic fatty liver disease (NAFLD) or a complication thereof.

The present disclosure also provides a method of treating non-alcoholic fatty liver disease (NAFLD) or a complication thereof, which comprises administering to a subject in need thereof a combination of lanifibranor (or a deuterated derivative thereof) and firsocostat.

In some embodiments, a deuterated derivative of lanifibranor is a compound of formula (I): 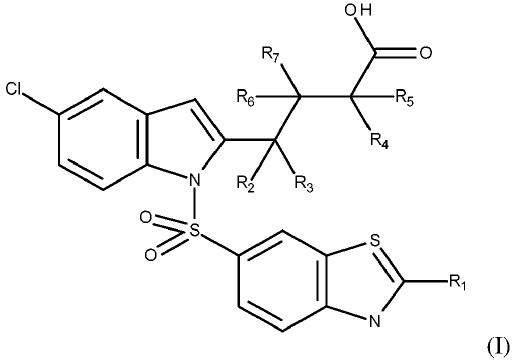 wherein at least one of the groups R₁ to R₇ is a deuterium (D) atom and the other groups R₁ to R₇ are hydrogen (H) atoms, as described in FR-A-3 084 254. In some aspects, at least group R₁ is D. In some aspects at least one of the groups R₂ to R₇ is D, notably at least one of the groups R₂ and R₃ and/or at least one of the groups R₄ and R₅ and/or at least one of the groups R₆ and R₇ is D. In a preferred aspect each of R₂, R₃, R₄, R₅, R₆ and R₇ is D. Preferred compounds of formula (I) include 4-(1-(2-deuterio-1,3-benzothiazol-6-yl)sulfonyl)-5-chloro-1H-indol-2-yl)butanoic acid and 4-[1-(1,3-benzothiazol-6-ylsulfonyl)-5-chloro-indol-2-yl]-2,2,3,3,4,4-hexadeuteriobutanoic acid.

In some embodiments, lanifibranor or a deuterated derivative thereof is in the form of one of its pharmaceutically acceptable salts or solvates. The term 'solvate' is used herein to describe a molecular complex comprising lanifibranor or a deuterated derivative thereof and one or more pharmaceutically acceptable solvent molecules, for example, ethanol. The term 'hydrate' is employed when said solvent is water. Pharmaceutically acceptable salts of lanifibranor or a deuterated derivative thereof include the acid and base addition salts thereof. Acid addition salts may be prepared from inorganic and organic acids. Examples of inorganic acids include hydrochloric acid, hydrobromic acid, sulfuric acid, nitric acid, phosphoric acid. Examples of organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, salicylic acid. Base addition salts may be prepared from inorganic and organic bases. Examples of inorganic bases include sodium hydroxide, potassium hydroxide, magnesium hydroxide and calcium hydroxide. Examples of organic bases include amines, amino alcohols, basic amino acids such as lysine or arginine, and quaternary ammonium compounds such as betaine or choline.

In some embodiments, firsocostat is in the form of one of its pharmaceutically acceptable salts, said salts being as defined herein.

Lanifibranor (or a deuterated derivative thereof) can be formulated into a pharmaceutical composition comprising one or more pharmaceutically acceptable excipients. The choice of excipient(s) will to a large extent depend on factors such as the particular mode of administration, the effect of the excipient on solubility and stability, and the nature of the dosage form. Pharmaceutical compositions can be prepared by conventional methods, as described e.g. in Remington's Pharmaceutical Sciences, 19th Edition (Mack Publishing Company, 1995), incorporated herein by reference.

In some embodiments, the pharmaceutical composition is suitable for oral administration. Examples of compositions suitable for oral administration include: (optionally coated) tablets, soft or hard (gelatin) capsules, lozenges, gels, syrups, or suspensions.

In some embodiments, the pharmaceutical composition comprises from about 100 to about 1200 mg of lanifibranor (or a deuterated derivative thereof), such as for example 100 mg, 200 mg, 300 mg, 400 mg, 500 mg, 600 mg, 700 mg, 800 mg, 900 mg, 1000 mg, 1100 mg or 1200 mg of said compound.

In some embodiments, lanifibranor (or a deuterated derivative thereof) is administered at a daily dose of from 200 mg to 1500 mg, such as for example a daily dose of 200 mg, 400 mg, 600 mg, 800 mg, 1000 mg, 1200 mg or 1500 mg. Lanifibranor (or a deuterated derivative thereof) can be administered once daily ("QD"), twice daily ("BID"), three time daily ("TID") or four times daily ("QID") provided the daily dose does not exceed the maximum amount indicated herein, i.e. 1500 mg.

In some embodiments, lanifibranor (or a deuterated derivative thereof) is administered to a subject with a meal. In some embodiments, lanifibranor (or a deuterated derivative thereof) is administered to a subject under fasted conditions.

Firsocostat can be formulated into a pharmaceutical composition comprising one or more pharmaceutically acceptable excipients. The choice of excipient(s) will to a large extent depend on factors such as the particular mode of administration, the effect of the excipient on solubility and stability, and the nature of the dosage form. Pharmaceutical compositions can be prepared by conventional methods, as described herein.

In some embodiments, the pharmaceutical composition is suitable for oral administration. Examples of compositions suitable for oral administration include: (optionally coated) tablets, soft or hard (gelatin) capsules, lozenges, gels, syrups, or suspensions.

In some embodiments, the pharmaceutical composition comprises from about 5 to about 200 mg of firsocostat, such as for example 5 mg, 10 mg, 20 mg, 50 mg, 100 mg or 200 mg of said compound.

In some embodiments, firsocostat is administered at a daily dose of from 10 mg to 200 mg, such as for example a daily dose of 10 mg, 20 mg, 40 mg, 80 mg, 100 mg or 200 mg. Firsocostat can be administered once daily ("QD"), twice daily ("BID"), three time daily ("TID") or four times daily ("QID") provided the daily dose does not exceed the maximum amount indicated herein, i.e. 200 mg.

In some embodiments, firsocostat is administered to a subject with a meal. In some embodiments, firsocostat is administered to a subject under fasted conditions.

In some embodiments, lanifibranor (or a deuterated derivative thereof) and firsocostat are administered simultaneously. In some embodiments, lanifibranor (or a deuterated derivative thereof) and firsocostat are administered sequentially. In some embodiment, lanifibranor (or a deuterated derivative thereof) and firsocostat are administered over a period of time.

In some embodiments, lanifibranor (or a deuterated derivative thereof) and firsocostat can be formulated into the same pharmaceutical composition comprising one or more pharmaceutically acceptable excipients. The pharmaceutical composition can be prepared as described herein. The respective amounts of lanifibranor (or a deuterated derivative thereof) and firsocostat in such a pharmaceutical composition are as described herein.

The invention is illustrated by the following, non-limiting example.

### Example

It has been reported (Duparc T et al., Am J Physiol Gasterointest Liver Physiol, 2019, Vol.317, n°4, pages G508-G517) that mice fed with a high fat/high cholesterol/high cholic acid diet with 2% 2-hydroxypropyl beta-cyclodextrin in drinking water for 3 weeks rapidly develops liver complications such as steatosis, inflammation and fibrosis with concomitant increase in plasma ALT/AST levels. The benefit of a combination therapy comprising lanifibranor and firsocostat was assessed in this model.

C57BL6/J mice were fed for 3 weeks with a 60% high fat / 1.25% cholesterol/0.5% cholic acid diet with 2% 2-hydroxypropyl beta-cyclodextrin in drinking water (HFCC/CDX diet). After 1 week of diet, blood was collected (-150 µL/ethylenediaminetetraacetic acid (EDTA)) in non-fasting conditions and plasma ALT and AST levels were measured. Mice were then randomized into 4 homogenous treatment groups (n=10 mice per group) according to their 1) ALT levels, 2) AST levels and 3) body weight. The four groups received then their treatment for the remaining 2 weeks on top of the HFCC/CDX diet as follows:
- Group 1: vehicle 1 + vehicle 2;
- Group 2: lanifibranor + vehicle 2;
- Group 3: vehicle 1 + firsocostat;
- Group 4: lanifibranor + firsocostat.

All treatments were given QD per os. Vehicle 1 stands for lanifibranor's vehicle (i.e. methyl cellulose/poloxamer), vehicle 2 stands for firsocostat's vehicle (i.e. Tween^{®}80/methylcellulose). Lanifibranor was given at 10mg/kg for 2 weeks and firsocostat was given at 30 mg/kg for the first 6 days and then at 15 mg/kg for the last 8 days: due to the high loss of weight observed in the combination group, the dose of firsocostat was adapted to limit toxicity. At the end of the treatment period all the mice were weighted and 4-hour fasted prior to blood collection (maximal volume/EDTA). Plasma was isolated and stored at -80°C. Plasma leftover was used for evaluation of plasmatic triglycerides, cholesterols and free fatty acid. After blood collection, mice were sacrificed by cervical dislocation under isoflurane anaesthesia and exsanguinated with sterile saline.

The liver was collected and weighted then liver samples were dissected for histology analysis (H&E, Sirius Red staining, % Sirius Red labelling and NAFLD Activity Score (NAS)), liver lipids assay, hepatic gene expression of IL-1b, MCP-1 for inflammation, Col1alpha1 and TGF-beta for fibrosis were analysed by qPCR.

### Body weight follow-up

As expected, HFCC+CDX diet did not impact the body weight during the two weeks of treatment. Lanifibranor induced a minor body weight loss that was not significant. Firsocostat however induced a body weight loss in the first week of treatment that was even more pronounced in the combination group when given at 30 mg/kg justifying a change in the dose that was reduced to 15 mg/kg. After this modification of dose both firsocostat and firsocostat + lanifibranor groups normalized their body weight (figure 1).

### Epididymal white adipose tissue

In the HFCC+CDX diet model, neither lanifibranor alone, firsocostat alone nor the combination of lanifibranor and firsocostat had an effect of the epididymal white adipose tissue (EWAT) weight (figure 2).

### Liver lipids

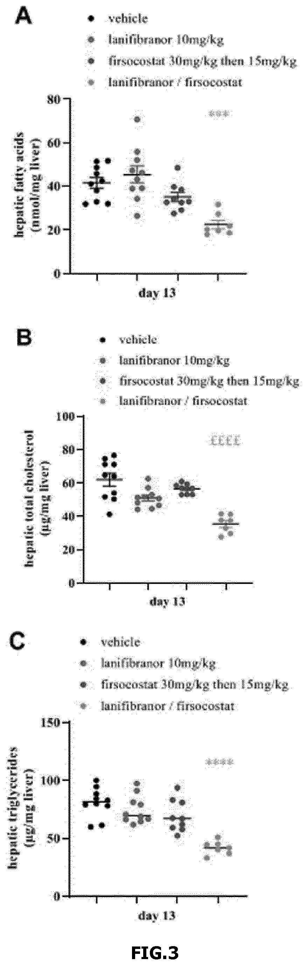

In the HFCC+CDX diet model, lanifibranor alone had no effect on hepatic fatty acids levels (figure 3A) and had a trend to decrease both hepatic total cholesterol (-18%) and hepatic triglycerides (-7%) however without reaching statistical significance (figure 3B and 3C). Firsocostat presented a trend in decreasing hepatic fatty acids levels (-16%), hepatic total cholesterol (-9%) and hepatic triglycerides (-14%) but without reaching statistical significance (figures 3A, 3B and 3C). The combination of lanifibranor and firsocostat decreased hepatic fatty acids levels (-46%; p<0.001 vs vehicle), hepatic total cholesterol (-43%; p<0.0001 vs vehicle) and hepatic triglycerides (-48% p<0.0001 vs vehicle) with a high significance (figures 3A, 3B and 3C).

### Hepatic inflammation gene expression

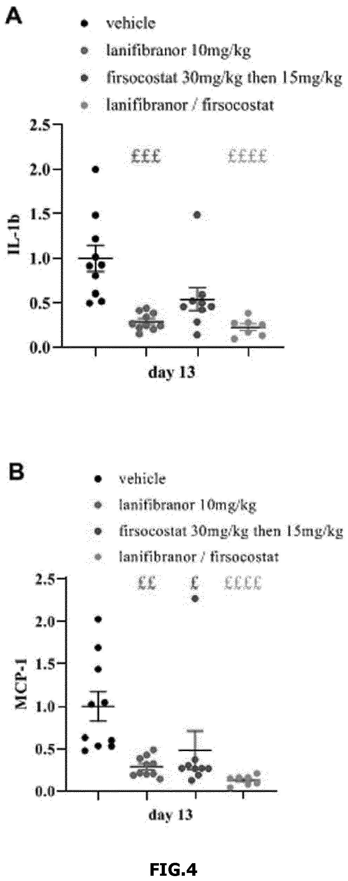

In the HFCC+CDX diet model, lanifibranor statistically decreased IL-1beta expression (p<0.001 vs vehicle) and MCP-1 expression (p<0.01 vs vehicle). Firsocostat also decreased both IL-1beta and MCP-1 expressions but only the decrease in MCP-1 expression reached significance (p<0.05 vs vehicle). The combination of lanifibranor and firsocostat further decreased the expression of IL-1beta and MCP-1 with a higher statistical significance (p<0.0001 vs vehicle, figures 4A and 4B).

### Hepatic fibrosis gene expression

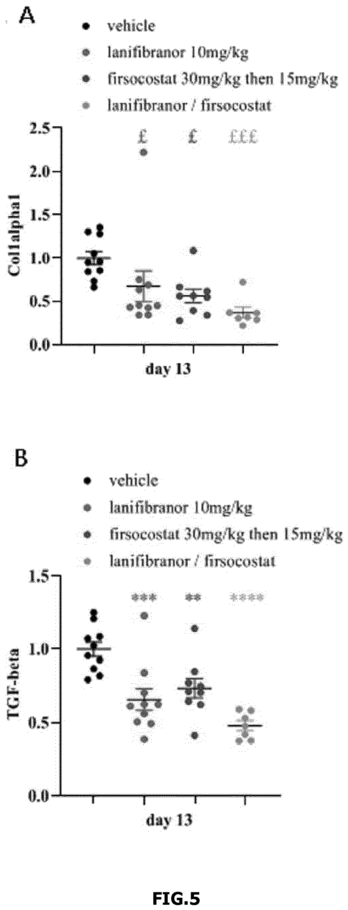

In the HFCC+CDX diet model, lanifibranor as well as firsocostat statistically decreased collagen 1alpha1 expression (p<0.05 vs vehicle) and TGF-beta1 expression (p<0.001 for lanifibranor and p<0.01 for firsocostat vs vehicle). The combination of lanifibranor and firsocostat further decreased the expression of collagen 1alpha1 and TGF-beta1 with a higher statistical significance (p<0.001 and p<0.01 vs vehicle respectively, figures 5A and 5B).

### Histological steatosis

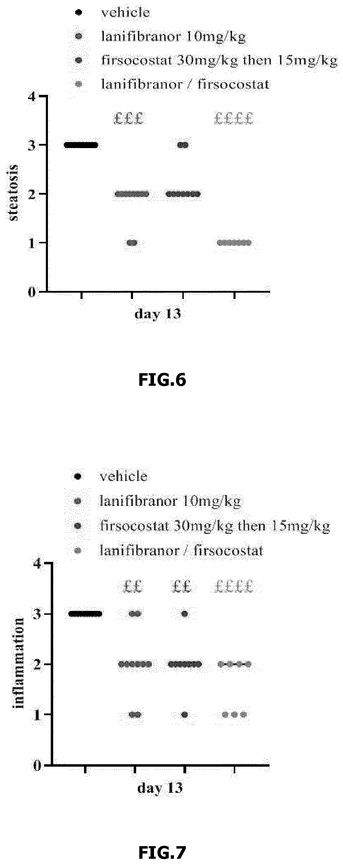

In the HFCC+CDX diet model, lanifibranor statistically (p<0.001 vs vehicle) decreased steatosis. All 10 vehicle animals presented a score of 3 (3, being the maximum) whereas the animals under lanifibranor had a score of 2 for 8 animals and of 1 for 2 animals. Firsocostat decreased the steatosis to a score of 2 in 7 animals but had no effect in 2 animals and consequently did not produce a significant effect. The combination of lanifibranor and firsocostat further decreased steatosis with a higher statistical significance (p<0.0001 vs vehicle) since all the animals presented a score of 1 (figure 6).

### Histological inflammation

In the HFCC+CDX diet model, lanifibranor as well as firsocostat statistically decreased inflammation (p<0.01 vs vehicle). All mice under vehicle treatment presented a score of 3 (3, being the maximum). Under lanifibranor treatment 2 animals had a score of 3, 6 animals had a score of 2 and 2 animals had a score of 1. Under firsocostat treatment 1 animals had a score of 3, 7 animals had a score of 2 and 1 animal had a score of 1. The combination of lanifibranor and firsocostat further decreased inflammation with a higher statistical significance (p<0.0001 vs vehicle): indeed none of the animals had a score of 3, 4 animals had a score of 2 and 3 animals had a score of 1 (figure 7).

### Histological fibrosis (scoring and % of fibrosis surface)

In the HFCC+CDX diet model, lanifibranor as well as firsocostat had no effect on the fibrosis score compared to the vehicle group but the combination of lanifibranor and firsocostat abolished the fibrosis in 5 mice out of 7 (p<0.01 vs vehicle). The two remaining mice had a score of 1 such as observed in the vehicle group (figure 8A).

In this model, lanifibranor as well as firsocostat also tended to decrease the surface of fibrosis (0.08% and 0.10% respectively, measured by the collagen deposition within the liver) compared to vehicle (0.14%) without being statistically significant. The combination of lanifibranor and firsocostat further decreased the fibrosis and demonstrated a statistically significant effect (0.06%, p<0.05 vs vehicle, figure 8B).

### Total score

In the HFCC+CDX diet model, lanifibranor as well as firsocostat statistically (p<0.01 for lanifibranor and p<0.05 for firsocostat vs vehicle) decreased the total scoring including steatosis, inflammation and fibrosis scoring (4.7 and 5.1 respectively) compared to vehicle (6.9). The combination of lanifibranor and firsocostat further decreased the total scoring (2.9) with a higher statistical significance compared to vehicle (p<0.0001 vs vehicle, figure 9).

The above results show that the combination of lanifibranor and firsocostat provides a beneficial effect in the mice treated, compared to lanifibranor alone and firsocostat alone, and is therefore suitable for the treatment of NAFLD and NASH. Markers such as liver lipids, steatosis, inflammation (histology and genes) and fibrosis (histology and genes) were improved to a greater extent with the combination than with lanifibranor alone and firsocostat alone.

### References

Mundi M S et al., Nutr Clin Pract 2020; Vol.35, n°1, pages 72-84
WO 2015/189401
EP-A-3 597 271
FR-A-3 084 254
Duparc T et al., Am J Physiol Gasterointest Liver Physiol. 2019, Vol.317, n°4, pages G508-G517

## Claims

1. A combination product comprising (i) lanifibranor or a deuterated derivative thereof, and (ii) firsocostat.

2. The combination product of claim 1, wherein lanifibranor or a deuterated derivative thereof is formulated in a first pharmaceutical composition and firsocostat is formulated in a second, distinct, pharmaceutical composition.

3. The combination product of claim 2, wherein the first pharmaceutical composition comprises from 100 mg to 1200 mg of lanifibranor or a deuterated derivative thereof.

4. The combination product of claim 2 or claim 3, wherein the second pharmaceutical composition comprises from 5 to 200 mg of firsocostat.

5. The combination product of claim 1, wherein lanifibranor or a deuterated derivative thereof and firsocostat are formulated in the same pharmaceutical composition.

6. The combination product of claim 5, wherein the pharmaceutical composition comprises from 100 mg to 1200 mg of lanifibranor or a deuterated derivative thereof, and from 5 to 200 mg of firsocostat.

7. A combination of (i) lanifibranor or a deuterated derivative thereof and (ii) firsocostat for use in a method of treating non-alcoholic fatty liver disease (NAFLD) or a complication thereof.

8. The combination for use of claim 7, whereon the complication of NAFLD is at least one of steatosis, steatohepatitis, non-alcoholic steatohepatitis (NASH), liver fibrosis caused by NASH, liver cirrhosis caused by NASH, liver failure caused by NASH, cardiovascular disease caused by NASH, or hepatocellular carcinoma caused by NASH.

9. The combination for use of claim 7 or claim 8, wherein lanifibranor or a deuterated derivative thereof is formulated in a first pharmaceutical composition and firsocostat is formulated in a second, distinct, pharmaceutical composition.

10. The combination for use of claim 7 or claim 8, wherein lanifibranor or a deuterated derivative thereof and firsocostat are formulated in the same pharmaceutical composition.

11. The combination for use of claim 7 or claim 8, wherein lanifibranor or a deuterated derivative thereof and firsocostat are administered simultaneously.

12. The combination for use of claim 7 or claim 8, wherein lanifibranor or a deuterated derivative thereof and firsocostat are administered sequentially.

13. The combination for use of claim 7 or claim 8, wherein lanifibranor or a deuterated derivative thereof and firsocostat are administered over a period of time.

14. A method of treating non-alcoholic fatty liver disease (NAFLD) or a complication thereof, which comprises administering to a subject in need thereof a combination of (i) lanifibranor or a deuterated derivative thereof and (ii) firsocostat.

15. The method of claim 14, whereon the complication of NAFLD is at least one of steatosis, steatohepatitis, non-alcoholic steatohepatitis (NASH), liver fibrosis caused by NASH, liver cirrhosis caused by NASH, liver failure caused by NASH, cardiovascular disease caused by NASH, or hepatocellular carcinoma caused by NASH.

16. The method of claim 14 or claim 15, wherein lanifibranor or a deuterated derivative thereof and firsocostat are administered simultaneously.

17. The method of claim 14 or claim 15, wherein lanifibranor or a deuterated derivative thereof and firsocostat are administered sequentially.

18. The method of claim 14 or claim 15, wherein lanifibranor or a deuterated derivative thereof and firsocostat are administered over a period of time.
